# EUROPÄISCHE PATENTANMELDUNG

(11) **EP 4 810 474 A1**
(43) Veröffentlichungstag der Anmeldung: **23.09.2026**
(21) Anmeldenummer: 25165266.5
(22) Anmeldetag: 21.03.2025
(51) Int. Cl.: C07C 45/74, C07C 49/603

(54) **FLÜSSIGPHASENVERFAHREN ZUR HERSTELLUNG VON ISOPHORON**

(71) Anmelder: Evonik Operations GmbH, 45128 Essen (DE)
(72) Erfinder: METTERNICH, Jan Benedikt, 45657 Recklinghausen (DE); NITZ, Jörg-Joachim, 45257 Essen (DE); WIESER, Melanie, 45897 Gelsenkirchen (DE); HANASEK, Maximilian, 45894 Gelsenkirchen (DE); KÜHNRICH, Ivo Robert, 44141 Dortmund (DE); LUDWIG, Martina, 45657 Recklinghausen (DE); MACKOWIAK, Dird, 46325 Borken (DE); ZÖLLNER, Jörg, 45657 Recklinghausen (DE); KOHLSTRUK, Stephan, 45966 Gladbeck (DE)
(74) Vertreter: Evonik Patent Association

(57) **Zusammenfassung**

Gegenstand der vorliegenden Erfindung ist ein Flüssigphasenverfahren zur Herstellung von Isophoron, bei dem Aceton katalysiert unter Zugabe von Mesityloxid kontinuierlich umgesetzt wird, wobei mindestens 50 Gew.-% des zugesetzten Mesityloxids nach Durchlauf von mindestens 50 % des Reaktorvolumens zugegeben wird.

## Beschreibung

Die vorliegende Erfindung betrifft ein Flüssigphasenverfahren zur Herstellung von Isophoron.

Isophoron ist eine bedeutende Industriechemikalie, deren Relevanz unter anderem darauf beruht, dass sie als Edukt für die Synthese von Isophoronnitril dient, das wiederum für die Synthese von als Epoxyhärter eingesetztem Isophorondiamin und des daraus wiederum herstellbaren Isophorondiisocyanats verwendet wird. Letzteres ist von großer Relevanz für die Polyurethanchemie. Aus diesem Grund sind Verfahren zur Herstellung von Isophoron von großem Interesse.

JP H09-157207 A und JP H09-157208 A offenbaren Verfahren zur Herstellung von Isophoron aus Aceton in der Gasphase. Die Reaktion wird in Gegenwart von Mesityloxid durchgeführt. Nachteilig an dem beschriebenen Verfahren ist, dass die Durchführung der Reaktion in der Gasphase hohe Temperaturen erfordern. Weiterhin ist nachteilig, dass die eingesetzten heterogenen Katalysatoren (Zeolithe) in regelmäßigen Abständen ausgetauscht und regeneriert werden müssen.

Diese Nachteile können dadurch überwunden werden, dass Isophoron basenkatalysiert in einem Flüssigphasenverfahren aus Aceton synthetisiert wird: DE 1 205 525 A1 offenbart ein Verfahren zur Herstellung von Isophoron in der flüssigen Phase, bei dem Aceton zu Isophoron kondensiert wird. Zu Entfernung von in beträchtlichen Mengen anfallendem Mesityloxid sowie von Überkondensaten können die Nebenprodukte mit wässriger Alkalilösung bei höheren Temperaturen behandelt werden. Es wird offenbart, dass Mesityloxid bei diesen Temperaturen vollständig abgebaut werde.

DE 26 45 281 A1 offenbart ein Flüssigphasenverfahren zur Herstellung von Isophoron, bei dem Aceton, Wasser und ein Alkalikatalysator umgesetzt werden. Das Isophoron wird destillativ aufgetrennt.

US 5,849,957 A offenbart ein Verfahren zur Herstellung von Isophoron aus Aceton in Gegenwart eines Magnesiumoxid-Katalysators. Entstehende Nebenprodukte schließen Mesityloxid ein und können recycelt werden.

US 2,419,051 A offenbart ein Verfahren zur Bereitstellung von Isophoron und Aceton, bei dem Autokondensationsprodukte von Aceton, die einen höheren Siedepunkt als Isophoron aufweisen, mit einem Alkalimetallhydroxid umgesetzt werden, um Isophoron und Aceton zu erzeugen.

GB 733,650 A offenbart, dass Aceton basenkatalysiert zu Isophoron und dem Zwischenprodukt Mesityloxid sowie höheren Kondensationsprodukten umgesetzt werden kann. In einer bevorzugten Ausführungsform werden die höheren Kondensationsprodukte in das Reaktionsgemisch zurückgeführt.

CN 103435461 A offenbart ein Verfahren zur Herstellung von Isophoron aus Aceton, bei dem nicht umgesetztes Aceton in die Reaktion zurückgeführt werden kann.

Auch DE 25 20 681 A1 offenbart ein Verfahren zur Herstellung von Isophoron, bei dem Aceton in Gegenwart von Kaliumhydroxid zu Isophoron umgesetzt wird. Entstehende Nebenprodukte können in der Hydrolyse zurück zu Aceton umgewandelt werden und werden der Reaktion wieder zugeführt.

CN 102367223 A offenbart ein Verfahren zur Herstellung von Isophoron, bei dem Aceton in Gegenwart eines Katalysators kondensiert wird. Nicht reagiertes Aceton wird abgetrennt und in die Reaktion zurückgeführt. Hochsieder mit 12 oder mehr Kohlenstoffatomen werden der Hydrolyse zugeführt.

US 2,344,226 A offenbart ein Verfahren zur Herstellung von Isophoron, bei dem Aceton in Gegenwart eines alkalischen Metallhydroxids erhitzt wird. Als Zwischenprodukt entstehendes Mesityloxid kann zusammen mit ebenfalls entstehenden Komponenten wie Diacetonalkohol, Phoron und höheren Kondensationsprodukten dem Reaktionsgemisch wieder zugegeben werden.

US 4,086,188 A offenbart Verfahren zur Herstellung von Mesityloxid und Isophoron. Es wird offenbart, dass es wünschenswert ist, dass das Verhältnis von gebildetem Mesityloxid zu gebildetem Isophoron kleiner als 1 ist, da anderweitig überschüssiges Mesityloxid recycliert werden muss.

DE 10 2010 062 587 A1 offenbart ein Flüssigphasenverfahren zur Herstellung von Isophoron aus Aceton, bei dem wässrige Alkalilauge als Katalysator verwendet werden kann. Die entstehenden Leichtsieder, umfassend Mesityloxid und Diacetonalkohol, können zusammen mit nicht umgesetzten Aceton in den Reaktor zurückgeführt werden.

Auch WO 2012/076317 A1 offenbart ein Flüssigphasenverfahren zur Herstellung von Isophoron aus Aceton, bei dem wässrige Alkalilauge als Katalysator eingesetzt werden kann. Auch hier können anfallende Leichtsieder, umfassend Mesityloxid und Diacetonalkohol, in die Reaktion zurückgeführt werden.

Es wurde festgestellt, dass bei der Aldolkondensation von Aceton zu Isophoron durch Zugabe von Mesityloxid, insbesondere durch Rückfuhr von im Reaktionsverlauf entstehenden Mesityloxid, die benötigten Mengen an Aceton vermindert werden konnten. Bei den bislang im Stand der Technik bekannten Verfahren wurde jedoch dabei der Umsatz bezogen auf Aceton, Mesityloxid und Diacetonalkohol vermindert, was nachteilig ist.

Es ist somit die Aufgabe der vorliegenden Erfindung, ein Flüssigphasenverfahren zur Herstellung von Isophoron aus Aceton unter Zufuhr von Mesityloxid bereitzustellen, mit dem höhere Umsätze erzielt werden können. Überraschenderweise wurde festgestellt, dass diese sich vorliegend stellende Aufgabe gelöst werden kann, wenn mindestens 50 Gew.-% des zugesetzten Mesityloxids nach Durchlauf von mindestens 50 % des Reaktorvolumens zugegeben wird.

Gegenstand der vorliegenden Erfindung ist somit ein Flüssigphasenverfahren zur Herstellung von Isophoron, bei dem Aceton in Gegenwart eines Katalysators unter Zugabe von Mesityloxid kontinuierlich umgesetzt wird, bei dem mindestens 50 Gew.-% des zugesetzten Mesityloxids nach Durchlauf von mindestens 50 % des Reaktorvolumens zugegeben wird.

Bei dem erfindungsgemäßen Verfahren handelt es sich um ein Flüssigphasenverfahren. Bei entsprechenden Verfahren wird die Reaktion in der flüssigen Phase durchgeführt. Das erfindungsgemäße Verfahren wird somit nicht in der Gasphase oder in überkritischem Aceton durchgeführt.

Bevorzugt wird die Reaktion bei Temperaturen von 100 - 250 °C, bevorzugt 120 - 250 °C, besonders bevorzugt 180 - 250 °C, und Drücken von 5 - 50 bar, bevorzugt 10 - 50 bar, besonders bevorzugt von 20 - 50 bar durchgeführt, wobei die angegebenen Werte beliebig miteinander kombiniert werden können.

Die Umsetzung erfolgt in Gegenwart eines Katalysators. Bei den eingesetzten Katalysatoren kann es sich um homogene oder heterogene Katalysatoren handeln. Bevorzugt wird ein homogener Katalysator eingesetzt.

Die Kondensation von Aceton zu Isophoron wird bevorzugt basisch katalysiert. Bevorzugt ist die eingesetzte Base eine Alkalilauge. Noch weiter bevorzugt wird der basische Katalysator ausgewählt aus der Gruppe bestehend aus NaOH und KOH.

Bevorzugt beträgt der Gehalt an Base, insbesondere an Alkalilauge, weiter bevorzugt NaOH oder KOH, kleiner oder gleich 5 Gew.-%, bevorzugt kleiner oder gleich 1 Gew.-%, besonders bevorzugt kleiner oder gleich 0,5 Gew.-%, bezogen auf die Gesamtmasse der Reaktionszusammensetzung. Ganz besonders bevorzugt wird als Katalysator NaOH in einer Menge von 0,005 bis 0,5 Gew.-% eingesetzt.

Insbesondere im Falle des Einsatzes wässriger Alkalilauge als Katalysator findet die Reaktion in Gegenwart von Wasser statt. Weiterhin entsteht im Laufe der Reaktion Wasser aufgrund des Kondensationsprozesses. Bevorzugt beträgt der Wassergehalt des Reaktionsgemisches, bestimmt über die zugeführte Wassermenge in den Reaktor, bezogen auf die zugeführten Mengen aus Aceton, Wasser und Mesityloxid, kleiner oder gleich 40 Gew.-%, bevorzugt kleiner oder gleich 30 Gew.-%.

Bevorzugt beträgt der Gehalt an Aceton im Gemisch, bestimmt über die zugeführte Acetonmenge in den Reaktor, bezogen auf die zugeführten Mengen aus Aceton, Wasser und Mesityloxid, größer oder gleich 25 Gew.-%, weiter bevorzugt größer oder gleich 50 Gew.-%, noch weiter bevorzugt größer oder gleich 65 Gew.-%.

Die Aldolkondensation von Aceton zu Isophoron erfolgt unter Zugabe von Mesityloxid. Bevorzugt beträgt der Gehalt an Mesityloxid, bezogen auf die zugeführten Mengen aus Aceton, Wasser und Mesityloxid, 0,1 - 50 Gew.-%, weiter bevorzugt 0,5 - 25 Gew.-%, noch weiter bevorzugt 0,8 - 10 Gew.-%.

Die Reaktion wird kontinuierlich in einem Reaktor durchgeführt. Unter einer kontinuierlichen Reaktionsführung ist vorliegend eine kontinuierlich erfolgende Zufuhr von Edukt und Katalysator in den Reaktor unter kontinuierlicher Ausfuhr von Produkt und nicht umgesetzten Edukten und Zwischenprodukten zu verstehen.

Bevorzugt für die kontinuierliche Reaktionsführung einsetzbare Reaktoren können ausgewählt werden aus der Gruppe bestehend aus Rohrreaktoren, Rührkesselkaskaden, Festbettreaktoren, Rieselbettreaktoren, Slurryreaktoren, Druckdestillationsreaktoren, Reaktivdestillationsreaktoren, mikrostrukturierten Reaktoren, Membranreaktoren, Kammerreaktoren, Statikmischern und Blasensäulen.

Bevorzugt handelt es sich bei dem Reaktor um einen Strömungsreaktor.

Erfindungsgemäß wird mindestens 50 Gew.-% des zugesetzten Mesityloxids nach Durchlauf von mindestens 50 % des Reaktorvolumens zugegeben. Unter dem Reaktorvolumen ist dabei das Volumen zu verstehen, innerhalb dessen Aceton in Gegenwart von Katalysator und mit sich und ggf. mit etwaigen vorhandenen Zwischenprodukten (einschließlich Mesityloxid) zu Isophoron umgesetzt wird. Weiter bevorzugt ist das Reaktorvolumen das Volumen des Reaktors, innerhalb dessen Aceton in Gegenwart von Katalysator mit sich und etwaig vorhandenen Zwischenprodukten (einschließlich Mesityloxid) bei einem Druck und einer Temperatur ausgewählt aus den bereits zuvor genannten bevorzugten Druck- und Temperaturbereichen miteinander in Kontakt gebracht werden. Insbesondere im Falle des Einsatzes eines Strömungsreaktors bzw. Rohrreaktors, dessen Querschnitt bzw. Rohr einen konstanten Durchmesser aufweist, entspricht die Position, an der x % des Reaktorvolumens durchlaufen wurde, x % der Länge des Reaktors.

Die Zugabe des Mesityloxids kann kontinuierlich erfolgen, muss aber nicht. Denkbar ist auch, dass zur Erzielung positiver Eigenschaften die Zugabe des Mesityloxids semikontinuierlich oder diskontinuierlich erfolgt. Bevorzugt wird das Mesityloxid jedoch ebenfalls kontinuierlich zugegeben.

Besonders hohe Umsätze können erzielt werden, wenn mindestens 60 Gew.-%, weiter bevorzugt mindestens 70 Gew.-%, noch weiter bevorzugt mindestens 75 Gew.-% des zugesetzten Mesityloxids nach Durchlauf von mindestens 50 % des Reaktorvolumens zugegeben wird.

Die erfindungsgemäß erfolgende Zugabe von mindestens 50 Gew.-%, bevorzugt mindestens 60 Gew.-%, weiter bevorzugt mindestens 70 Gew.-%, noch weiter bevorzugt mindestens 75 Gew.-% des Mesityloxids nach Durchlauf von mindestens 50 % des Reaktorvolumens kann über eine Dosierstelle oder zwei oder mehr Dosierstellen, deren Position sich sämtlich nach Durchlauf von mindestens 50 % des Reaktorvolumens befinden, erfolgen. Bevorzugt erfolgt die Zugabe von mindestens 50 Gew.-%, weiter bevorzugt mindestens 60 Gew.-%, noch weiter bevorzugt mindestens 70 Gew.-%, ganz besonders bevorzugt mindestens 75 Gew.-% des Mesityloxids nach Durchlauf von mindestens 50 % des Reaktorvolumens über 2 oder mehr, weiter bevorzugt 3 oder mehr, ganz besonders bevorzugt 4 oder mehr Dosierstellen, deren Position sich sämtlich nach Durchlauf von mindestens 50 % des Reaktorvolumens befinden. Eine bevorzugte Obergrenze der Zahl dieser Dosierstellen beträgt bevorzugt 20, weiter bevorzugt 15, noch weiter bevorzugt 10.

Die restlichen, an 100 Gew.-% fehlenden Prozentanteile des zugesetzten Mesityloxids (für mindestens 50 Gew.-% nach 50 % des Reaktorvolumens zugegebenen Mesityloxids also kleiner oder gleich 50 Gew.-%, für mindestens 60 Gew.-% nach 50 % des Reaktorvolumens zugegebenen Mesityloxids also kleiner oder gleich 40 Gew.-%, für mindestens 70 Gew.-% nach 50 % des Reaktorvolumens zugegebenen Mesityloxids also kleiner oder gleich 30 Gew.-%, für mindestens 75 Gew.-% nach 50 % des Reaktorvolumens zugegebenen Mesityloxids also kleiner oder gleich 25 Gew.-%) werden vor Durchlauf von mindestens 50 % des Reaktorvolumens zugegeben. Auch hier kann die Zugabe über eine oder mehr, bevorzugt 2 oder mehr, weiter bevorzugt 3 oder mehr Dosierstellen erfolgen. Eine bevorzugte Obergrenze der Zahl dieser Dosierstellen beträgt bevorzugt 15, weiter bevorzugt 10, noch weiter bevorzugt 5.

Bevorzugt erfolgt keine Zugabe von Mesityloxid vor dem Durchlauf von mindestens 25 % des Reaktorvolumens, da so besonders gute Umsätze erzielt werden.

Bevorzugt erfolgt keine Zugabe von Mesityloxid vor dem Durchlauf von mindestens 50 % des Reaktorvolumens, da so besonders gute Umsätze in Kombination mit besonders guten Selektivitäten erzielt werden.

Bevorzugt stammt das zugeführte Mesityloxid aus der Aufarbeitung des Wertstoff-haltigen Reaktionsgemisches des Flüssigphasenverfahrens zur Herstellung von Isophoron.

Das Wertstoff-haltige Reaktionsgemisch nach dem Durchlauf des gesamten Reaktorvolumens kann dafür aufgearbeitet und in die einzelnen Komponenten getrennt werden. Dabei fallen neben Isophoron Aceton, die Leichtsieder (d.h. Stoffe mit einem niedrigeren Siedepunkt als Isophoron, insbesondere Diacetonalkohol und Mesityloxid), höhere Kondensationsprodukte (Überkondensate) des Acetons (d.h. mit einem höheren Siedepunkt als Isophoron, insbesondere Xylitone und Isoxylitone), Wasser und Katalysator an.

Die Auftrennung in die einzelnen Komponenten kann prinzipiell mit allen Trennmethoden, insbesondere Destillation, Entspannungsverdampfung, Kristallisation, Extraktion, Sorption, Permeation, Phasentrennung oder Kombinationen davon kontinuierlich oder absatzweise, ein- oder mehrstufig durchgeführt werden. Bevorzugt erfolgt die Trennung durch Destillation in einem oder mehreren Apparaten.

Weiter bevorzugt erfolgt die Abtrennung der einzelnen Fraktionen durch eine auf den Reaktor folgende Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Bevorzugt wird die Abtrennung so durchgeführt, dass die folgenden drei Fraktionen erhalten werden:
i) Eine Fraktion aus nicht umgesetzten Aceton, Wasser und Leichtsiedern (insbesondere Diacetonalkohol und Mesityloxid)
ii) eine Fraktion, in der insbesondere farbgebende Substanzen angereichert werden, und
iii) eine Fraktion umfassend insbesondere Isophoron, höherkondensierte Produkte, Wasser und Katalysator, genannt Wertstrom. Diese Fraktion wird anschließend einer Hydrolyse unterworfen.

Bislang wurde die Fraktion i) bevorzugt kondensiert und anschließend zur Reaktion in den Reaktor zurückgeführt. In einer weiter bevorzugten Ausführungsform wurde sie als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.

In einer bevorzugten Ausführungsform der nun vorliegenden Erfindung erfolgt die Zugabe des Mesityloxids über die Zugabe einer Fraktion umfassend Aceton, Wasser und Leichtsieder, die über eine Destillation des Wertstoff-haltigen Reaktionsgemischs nach dem Durchlauf des gesamten Reaktorvolumens erhältlich ist. Prinzipiell kann somit die Zugabe des Mesityloxids zusammen mit anderen Leichtsiedern, insbesondere Diacetonalkohol, sowie Wasser und Aceton erfolgen. Etwaige anwesende, sich von Mesityloxid unterscheidende Leichtsieder finden dabei keine Berücksichtigung bei der Bestimmung des Prozentgehaltes an Mesityloxid, d.h. der angegebene Prozentwert bezieht sich immer auf die zugeführten Mengen an Aceton, Wasser und Mesityloxid. Weiter bevorzugt entspricht diese Fraktion somit der o.g. Fraktion i).

Bevorzugt weist die Fraktion Anteile von 60 - 85 Gew.-% Aceton, 10 - 25 Gew.-% Wasser und, 0.5 - 10 Gew.-% Diacetonalkohol und 0.5 - 10 Gew.-% Mesityloxid auf.

Weiter bevorzugt wird
- aus der Aceton, Wasser und Leichtsieder umfassenden Fraktion, bevorzugt der Fraktion i),
   o Aceton und Wasser von den Leichtsiedern
      ▪ abgetrennt, bevorzugt destillativ abgetrennt, und
      ▪ zu Anfang des Reaktors zugegeben.

Bei dieser ebenfalls bevorzugten Ausführungsform erfolgt die Zugabe des Mesityloxids somit über die Zugabe der Leichtsieder, bevorzugt bestehend im Wesentlichen aus einem Gemisch aus Mesityloxid und Diacetonalkohol, die über
- eine Destillation des Wertstoff-haltigen Reaktionsgemischs nach dem Durchlauf des gesamten Reaktorvolumen des Reaktors unter Erhalt einer Aceton, Wasser und Leichtsieder umfassenden Fraktion und
- nachfolgende Abtrennung, bevorzugt destillative Abtrennung, von Aceton und Wasser aus der genannten Fraktion zugänglich sind.

Die Fraktion ii) wird in einer bevorzugten Ausführungsform als Seitenstrom der Destillationskolonne, bevorzugt einer Reaktivdestillationskolonne, entnommen, gegebenenfalls neutralisiert und weiter aufgearbeitet. Diese Fraktion kann weiter aufgereinigt und die enthaltenden Wertstoffe in den Prozess zurückgeführt werden. Bei der Aufarbeitung können alle gängigen Trennmethoden wie z. B. Destillation, Entspannungsverdampfung, Kristallisation, Extraktion, Sorption, Permeation, Phasentrennung oder Kombinationen davon zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, bevorzugt in die Reaktion zurückgeführt werden. Bevorzugt erfolgt dies jedoch nicht, da der apparative Aufwand zu groß ist. Gegebenenfalls anfallende Rückstände können der thermischen Verwertung zugeführt werden.

Die Fraktion iii) fällt bevorzugt im Sumpf der Destillationskolonne an und kann von dort unter Erhalt des Wertstroms entnommen werden.

Der Wertstrom der Aldolkondensation wird nachfolgend bevorzugt b) hydrolysiert, d.h. einer Hydrolyse unterworfen. Das Ziel der Hydrolyse ist es, im Wertstrom enthaltene Nebenprodukte teilweise oder vollständig in Isophoron, Aceton, oder andere nutzbare Wertprodukte zu überführen. Die Hydrolyse kann in allen gängigen Reaktoren, Destillationskolonnen oder Kombinationen davon durchgeführt werden. Bevorzugt wird die Hydrolyse als Reaktivdestillation durchgeführt, in dem die entstehenden Leichtsieder, im Wesentlichen bestehend aus Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt kann die Hydrolyse der Fraktion iii) in einem Apparat durch eine Reaktivdestillation erfolgen, bevorzugt in einer Reaktivdestillationskolonne, wobei gleichzeitig die Trennung des Reaktionsgemisches in die Fraktionen i) bis iii) erfolgt, so dass die entstehenden Produkte gleichzeitig entsprechend aufgetrennt werden und die Fraktion iii) hydrolysiert wird.

Die Hydrolyse kann in allen Mischungsverhältnissen der organischen Komponenten mit Wasser mit oder ohne Katalysator durchgeführt werden. Die Wasserkonzentration in der Hydrolyse beträgt 30,1 - 99.9 Gew.-% bezogen auf die Gesamtmasse der anwesenden Bestandteile. Im Falle der homogenen Katalyse wird bei der Hydrolyse bevorzugt derjenige Katalysator eingesetzt, der auch bei der Aldolkondensation verwendet wird. Bevorzugt werden Katalysatorkonzentrationen von 0,001 - 10 Gew.-%, besonders bevorzugt von 0,05 - 1 Gew.-%, bezogen auf die Gesamtmasse der Zusammensetzung, gewählt. Der Druck im Hydrolysereaktor beträgt 1-200 bar, bevorzugt 20 - 60 bar. Besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse kann in einem oder mehreren Apparaten einstufig oder mehrstufig durchgeführt werden.

Das erhaltene Hydrolysat wird anschließend bevorzugt, nachdem es weiter bevorzugt aus dem Hydrolysereaktor der Reaktivdestillationskolonne ausgeführt und abgekühlt wurde, einer Phasentrennung c) unterworfen.

Durch die Phasentrennung kann eine im Wesentlichen organische Fraktion i) und eine im Wesentlichen wässrige Fraktion ii) erhalten werden. Im Falle einer homogenen Katalyse der Aldolkondensation enthält die wässrige Fraktion ii) auch den Katalysator. Für die Phasentrennung können übliche Phasentrennbehälter mit oder ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C, und bei einem Druck von 1 - 150 bar, bevorzugt 20 - 60 bar, besonders bevorzugt bei dem Druck, der auch in der Hydrolyse herrscht.

Die im Wesentlichen organische Phase i) umfassend das Zielprodukt Isophoron kann ggf. neutralisiert und mit üblichen Methoden aufgereinigt werden, so dass ein Isophoron mit der gewünschten Reinheit und Farbstabilität erhalten wurde. Dabei können alle gängigen Trennmethoden, insbesondere Destillation, Entspannungsverdampfung, Kristallisation, Extraktion, Sorption, Permeation, Phasentrennung, sowie Kombinationen davon zum Einsatz kommen. Die Aufreinigung kann kontinuierlich oder absatzweise, ein- oder mehrstufig, unter Druck oder im Vakuum durchgeführt werden. Bevorzugt wird die Aufreinigung durch Destillation erreicht. Besonders bevorzugt wird die Aufreinigung durch eine Kombination aus Neutralisation oder Extraktion und nachfolgender Destillation erreicht.

Die im Wesentlichen wässrige Fraktion ii) kann einer Abwasseraufreinigung zugeführt werden. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten. Die Abwasseraufreinigung wird bevorzugt in einer oder mehreren Destillationskolonnen durchgeführt.

Das mit dem erfindungsgemäßen Verfahren herstellbare Isophoron eignet sich für eine Vielzahl von Anwendungen. Insbesondere eignet es sich für die Herstellung von Isophoronnitril. Isophoronnitril lässt sich weiterhin vorteilhaft einsetzen für die Synthese von Isophorondiamin, insbesondere über aminierende Hydrierung. Isophorondiamin lässt sich weiterhin zu Isophorondiisocyanat umsetzen.

Gegenstand der vorliegenden Erfindung ist somit nicht nur das mit dem erfindungsgemäßen Verfahren erhältliche Isophoron, sondern auch seine Verwendung zur Herstellung von Isophoronnitril, Isophorondiamin und/oder Isophorondiisocyanat.

Alle angegebenen Prozent (%) sind, wenn nicht anders angegeben, Massenprozent.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

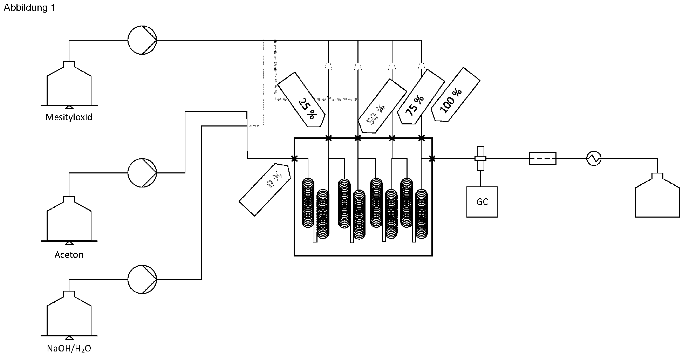

Die Experimente wurde in einer kontinuierlich betriebenen Laboranlage durchgeführt (siehe Abbildung 1). Die Anlage bestand aus Vorlagen für Aceton, wässriger Natronlaugelösung und Mesityloxid, Pumpen zur Dosierung der Edukte, einem mittels Thermalöl beheizten Rohrreaktor, einem Nachdruckregler, sowie Online-GC und Probenahmestelle für Offline-Untersuchungen. Die Dosierung der Feeds erfolgte über HPLC-Pumpen in einem Volumenstrombereich von 0.4 - 5.4 mL/min. Der Rohrreaktor wies ein Volumen von 235 mL auf und wurde zusammengesetzt aus 16 separaten Wendeleinheiten mit einer Gesamtzahl von 256 Wicklungen. Effiziente Durchmischung wurde durch Inversion der Strömungsrichtung nach je 6.25 % des Reaktorvolumens erzielt. Die Dosierung von Mesityloxid in den Reaktor erfolgte über T-Stücke nach 0%, 25%, 50%, 75% und 100% des Reaktorvolumens. Zur genauen Kontrolle des Volumenstroms wurde die Zugabe über geeignete Restriktoren vorgenommen. Analytische Betrachtungen des Reaktionsfortschrittes erfolgte mittels Online-Gaschromatographie unter Verwendung eines druckstabilen Stempeldosierers. Die Verifizierung der Online-Messungen wurde durch Entnahme von Probenmaterial nach erfolgter Reaktion und Untersuchung mittels Offline-Gaschromatographie erzielt.

### 1. Herstellung von Isophoron durch basenkatalysierte Reaktion von Aceton mit Zugabe von Mesityloxid bei 0% des Reaktorvolumens

Aceton (5,39 mL/min) und wässrige Natronlauge (0,169 Gew.-% in Wasser, 1.42 mL/min) wurden vor dem Reaktor zusammengeführt. Mesityloxid (0,42 mL/min, 6 Gew.-% des Gesamtfeeds) wurde bei 0 % vollständig zudosiert. Die Umsetzung erfolgte im Rohrreaktor bei 200 °C, 35 bar und einer resultierenden, mittleren Verweilzeit von 35 Minuten. Der Umsatz bezogen auf Aceton, Mesityloxid und Diacetonalkohol betrug 8,0 % und die Selektivität für Isophoron 89,6 %.

### 2. Herstellung von Isophoron durch basenkatalysierte Reaktion von Aceton mit Zugabe von Mesityloxid bei 50% des Reaktorvolumens

Aceton (5,39 mL/min) und wässrige Natronlauge (0,169 Gew.-% in Wasser, 1,42 mL/min) wurden vor dem Reaktor zusammengeführt. Mesityloxid (0,42 mL/min, 6 Gew.-% des Gesamtfeeds) wurde nach 50 % des Reaktorvolumens vollständig zudosiert. Die Umsetzung erfolgte im Rohrreaktor bei 200 °C, 35 bar und einer resultierenden, mittleren Verweilzeit von 35 Minuten. Der Umsatz bezogen auf Aceton, Mesityloxid und Diacetonalkohol betrug 8,4 % und die Selektivität für Isophoron 89,5%.

### 3. Herstellung von Isophoron durch basenkatalysierte Reaktion von Aceton mit Zugabe von Mesityloxid bei 25%, 50%, 75% und 100% des Reaktorvolumens

Aceton (5,39 mL/min) und wässrige Natronlauge (0,169 Gew.-% in Wasser, 1,42 mL/min) wurden vor dem Reaktor zusammengeführt. Mesityloxid (gesamt: 0,42 mL/min, 6 Gew.-% des Gesamtfeeds) wurde nach 25 %, 50 %, 75 % und 100 % (je 0,11 mL/min, 1,5 Gew.-% des Gesamtfeeds) des Reaktorvolumens schrittweise zudosiert. Die Umsetzung erfolgte im Rohrreaktor bei 200 °C, 35 bar und einer resultierenden, mittleren Verweilzeit von 35 Minuten. Der Umsatz bezogen auf Aceton, Mesityloxid und Diacetonalkohol betrug 8,6 % und die Selektivität für Isophoron 86,4 %.

## Patentansprüche

1. Flüssigphasenverfahren zur Herstellung von Isophoron, bei dem
Aceton in Gegenwart eines Katalysators unter Zugabe von Mesityloxid kontinuierlich umgesetzt wird,
**dadurch gekennzeichnet, dass**
mindestens 50 Gew.-% des zugesetzten Mesityloxids nach Durchlauf von mindestens 50 % des Reaktorvolumens zugegeben wird.

2. Verfahren nach Anspruch 1,
**dadurch gekennzeichnet, dass**
die Umsetzung bei Temperaturen von 100 - 250 °C und einem Druck von 5 - 50 bar erfolgt.

3. Verfahren nach Anspruch 1 oder 2,
**dadurch gekennzeichnet, dass**
der Katalysator ausgewählt wird aus NaOH und KOH.

4. Verfahren nach einem der vorhergehenden Ansprüche,
**dadurch gekennzeichnet, dass**
der Reaktor ausgewählt wird aus der Gruppe an Reaktoren bestehend aus Rohrreaktoren, Rührkesselkaskaden, Festbettreaktoren, Rieselbettreaktoren, Slurryreaktoren, Druckdestillationsreaktoren, Reaktivdestillationsreaktoren, mikrostrukturierten Reaktoren, Membranreaktoren, Kammerreaktoren, Statikmischern und Blasensäulen.

5. Verfahren nach einem der vorhergehenden Ansprüche,
**dadurch gekennzeichnet, dass**
der Reaktor ein Strömungsreaktor ist.

6. Verfahren nach einem der vorhergehenden Ansprüche,
**dadurch gekennzeichnet, dass**
mindestens 60 Gew.-% des zugesetzten Mesityloxids nach Durchlauf von mindestens 50 % des Reaktorvolumens zugegeben wird.

7. Verfahren nach einem der vorhergehenden Ansprüche,
**dadurch gekennzeichnet, dass**
die Zugabe der mindestens 50 Gew.-% des Mesityloxids nach Durchlauf von mindestens 50 % des Reaktorvolumens über zwei oder mehr Dosierstellen, deren Position sich sämtlich nach Durchlauf von mindestens 50 % des Reaktorvolumens befinden, erfolgt.

8. Verfahren nach einem der vorhergehenden Ansprüche,
**dadurch gekennzeichnet, dass**
keine Zugabe von Mesityloxid vor dem Durchlauf von mindestens 25 % des Reaktorvolumens erfolgt.

9. Verfahren nach Anspruch 8,
**dadurch gekennzeichnet, dass**
keine Zugabe von Mesityloxid vor dem Durchlauf von mindestens 50 % des Reaktorvolumens erfolgt.

10. Verfahren nach einem der vorhergehenden Ansprüche,
**dadurch gekennzeichnet, dass**
das zugeführte Mesityloxid aus der Aufarbeitung des Wertstoff-haltigen Reaktionsgemisches des Flüssigphasenverfahrens stammt.

11. Verfahren nach Anspruch 10,
**dadurch gekennzeichnet, dass**
die Zugabe des Mesityloxids
über die Zugabe einer Fraktion umfassend Aceton, Wasser und Leichtsieder, die über eine Destillation des Wertstoff-haltigen Reaktionsgemischs nach dem Durchlauf des gesamten Reaktorvolumen erhältlich ist, erfolgt.

12. Verfahren nach Anspruch 10,
**dadurch gekennzeichnet, dass**
die Zugabe des Mesityloxids
über die Zugabe der Leichtsieder, die über
- eine Destillation des Wertstoff-haltigen Reaktionsgemischs nach dem Durchlauf des gesamten Reaktorvolumens unter Erhalt einer Aceton, Wasser und Leichtsieder umfassenden Fraktion und
- nachfolgende Abtrennung von Aceton und Wasser aus der genannten Fraktion zugänglich sind, erfolgt.
